# EUROPEAN PATENT SPECIFICATION

(11) **EP 3 548 105 B1**
(45) Date of publication and mention of the grant of the patent: **05.03.2025**
(21) Application number: 17822782.3
(22) Date of filing: 01.12.2017
(51) Int. Cl.: A61L 27/22, A61L 27/54, A61L 27/34

(54) **METHOD FOR PRODUCING A PEPTIDE-BASED NANO FIBER STRUCTURE AND A NANO FIBER STRUCTURE THUS OBTAINED**
VERFAHREN ZUR HERSTELLUNG EINES PEPTIDBASIERTEN NANOFASERS UND EIN DAMIT HERGESTELLTER NANOFASER
PROCÉDÉ POUR LA PRÉPARATION D'UNE NANOFIBRE À BASE DE PEPTIDES ET LA NANOFIBRE AINSI OBTENUE

(30) Priority: 02.12.2016 IT 201600116974
(43) Date of publication of application: 09.10.2019
(73) Proprietor: Humana Medical S.r.l., 35121 Padova (IT)
(72) Inventor: ORNAGHI, Oscar Marco, 35026 Montegrotto Terme (PD) (IT); BINOTTO, Radames, 35038 Torreglia (PD) (IT); MODESTI, Michele, 35127 Padova (IT); ROSO, Martina, 37142 Verona (IT)
(74) Representative: Susanetto, Carlo
(86) International application number: PCT/IB2017/057572
(87) International publication number: WO 2018/100546

(56) References cited:
- ANN C STIJNMAN ET AL: "Electrospinning of food-grade polysaccharides", FOOD HYDROCOLLOIDS, ELSEVIER BV, NL, vol. 25, no. 5, 7 January 2011 (2011-01-07), pages 1393 - 1398, XP028163291, ISSN: 0268-005X, [retrieved on 20110118], DOI: 10.1016/J.FOODHYD.2011.01.005
- SUNTHORNVARABHAS, J ET AL.: "Physical structure behavior to wettability of electrospun poly(lactic acid)/polysaccharide composite nanofibers", ADVANCED COMPOSITE MATERIALS, vol. 22, 1 December 2013 (2013-12-01), pages 401 - 409
- YE XINGUO ET AL: "Pectin based composite nanofabrics incorporated with layered silicate and their cytotoxicity", INTERNATIONAL JOURNAL OF BIOLOGICAL MACROMOLECULES, ELSEVIER BV, NL, vol. 93, 18 August 2016 (2016-08-18), pages 123 - 130, XP029790763, ISSN: 0141-8130, DOI: 10.1016/J.IJBIOMAC.2016.08.047
- HU J ET AL: "Electrospun Poly(N-isopropylacrylamide)/Ethyl Cellulose Nanofibers as Thermoresponsive Drug Delivery Systems", JOURNAL OF PHARMACEUTICAL SCIENCES, vol. 105, no. 3, 1 March 2016 (2016-03-01), DOI: https://dx.doi.org/10.1016/S0022-3549(15)00191-4
- FAZEL RAMIN ET AL: "Electrospun polyvinyl alcohol/bovine serum albumin biocomposite membranes for horseradish peroxidase immobilization", ENZYME AND MICROBIAL TECHNOLOGY, STONEHAM, MA, US, vol. 93, 10 July 2016 (2016-07-10), pages 1 - 10, XP029756626, ISSN: 0141-0229, DOI: 10.1016/J.ENZMICTEC.2016.07.002
- ZHAO W ET AL: "Preparation of animal polysaccharides nanofibers by electrospinning and their potential biomedical applications", JOURNAL OF BIOMEDICAL MATERIALS RESEARCH PART A, vol. 103, 15 April 2014 (2014-04-15), pages 807 - 818
- LIANG ET AL: "Functional electrospun nanofibrous scaffolds for biomedical applications", ADVANCED DRUG DELIVERY REVIEWS, ELSEVIER, AMSTERDAM , NL, vol. 59, no. 14, 22 November 2007 (2007-11-22), pages 1392 - 1412, XP022356814, ISSN: 0169-409X, DOI: 10.1016/J.ADDR.2007.04.021
- KUEN YONG LEE ET AL: "Electrospinning of polysaccharides for regenerative medicine", ADVANCED DRUG DELIVERY REVIEWS, vol. 61, no. 12, 1 October 2009 (2009-10-01), pages 1020 - 1032, XP055009067, ISSN: 0169-409X, DOI: 10.1016/j.addr.2009.07.006
- R GHARAEI ET AL.: "A structurally self-assembled peptide nano-architecture by one-step electrospinning", JOURNAL OF MATERIALS CHEMISTRY B, vol. 4, 25 July 2016 (2016-07-25), Royal Society of Chemistry, pages 5475 - 5485, XP002774050, ISSN: 0959-9428
- P BRUN ET AL.: "Electrospun scaffolds of self-assembling peptides with poly(ethylene oxide) for bone tissue engineering", ACTA BIOMATERIALIA, vol. 7, no. 6, 15 February 2011 (2011-02-15), ELSEVIER, pages 2526 - 2532, XP028199371, ISSN: 1742-7061
- M DETTIN ET AL.: "Electrospun scaffolds for osteoblast cells: peptide-induced concentration-dependent improvements of polycaprolactone", PLOS ONE, vol. 10, no. 9, 11 September 2015 (2015-09-11), Public Library of Science, pages 1 - 23, XP002774051, ISSN: 1932-6203, DOI: 10:13717journal.pone.0137505
- A ANDUKURI ET AL.: "A hybrid biomimetic nanomatrix composed of electrospun polycaprolactone and bioactive peptide amphiphiles for cardiovascular implants", ACTA BIOMATERIALIA, vol. 7, no. 1, 1 January 2011 (2011-01-01), ELSEVIER, pages 225 - 233, XP027448764, ISSN: 1742-7061

## Description

### Technical scope

The present invention relates to a method for producing a peptide-based nanofibre structure.

Preferably, although not exclusively, this structure can be applied in the medical sector, particularly in therapeutic treatments to promote tissue regeneration.

The invention also relates to a peptide-based nanofibre structure obtained by that method.

### Background Art

The use of biomolecules, particularly peptides, has in recent years occupied ever-larger space in various fields of technology, including, in addition to the medical and bioengineering field. also the electronics and information technology field.

The medical sector, however, certainly remains the technical field in which such compounds arouse greatest interest and where they are used most successfully.

Significant examples that can be mentioned are applications of peptides as carriers for administering drugs, in which peptides are used for introducing the active substance of the drug directly into the cells, or applications of peptides in therapeutic treatments for tissue or bone regeneration, including healing epithelial lacerations.

In recent years, particular attention has been paid to self-assembling peptides (indicated below either in full or abbreviated to SAP), a specific class of peptides that tend to assemble together spontaneously when placed in specific environments, for example in physiological conditions or in a solution containing monovalent cations.

SAPs are oligopeptides formed of a chain of amino-acids numbering between 5 and 35 and they comprise amino-acids having hydrophobic residues variously alternating with amino-acids having hydrophilic residues. These oligopeptides, when in their natural conformation, for example a β (beta)-sheet, tend to arrange the hydrophilic residues on one side and the hydrophobic residues on the other, forming a hydrophilic side and a hydrophobic side. Under the above-mentioned conditions, SAPs therefore tend to be superimposed on one another with the respective hydrophobic sides and the respective hydrophilic sides facing each other, forming a nanometric structure stabilised by ionic, Van der Waals interactions, hydrogen bonds, created between the sides of the oligopeptides.

Because of this specific attribute, SAPs are also known as "molecular Lego".

SAPs can easily be functionalised by inserting, into their oligopeptide chain, groups that can stimulate cell growth and/or cell differentiation (for example growth factors) or can promote cell adhesion (for example cell adhesion molecules - CAMs), or else can help to attract specific compounds (for example chemotactic factors).

These properties make them particularly suitable for use in therapeutic treatments for tissue or bone regeneration.

In this latter type of treatment, it is known that the regeneration process is assisted by the presence *in situ* of structures that tend to reproduce the natural extracellular matrix as exactly as possible.

This type of structure acts as a sort of "scaffold", as it is generally known in the sector, capable of supporting the cell architecture.

The correct design of this structure (scaffold) plays a decisive part in the efficacy of the therapeutic treatment.

Said structure shall, in fact, have mechanical, chemical and biological properties that mimic the natural extracellular matrix, interacting properly with both the tissue and the surrounding cells, so as to allow and, still better, to promote the natural processes of forming the new organic tissue.

In particular, it is preferable and, in some cases, necessary for such structures to meet the following requirements.

Firstly, they shall be produced from biocompatible, non-toxic material so as not to damage the parts of the body with which they come into contact and so as not to inhibit the formation of new cells.

Furthermore, it is preferable for them to be made of bioabsorbable material so that, in due course, they can gradually degrade within the body; this avoids having to keep a foreign body in the body indefinitely or even having to remove it in a surgical procedure.

Moreover, they shall be porous enough to encourage growth and cellular diffusion and allow a free flow of substances from and to the cells. Furthermore, they shall have suitable mechanical properties, depending also on the specific tissue to be regenerated.

Moreover, they shall have a large surface area and, preferably, superficial chemical and physical properties promoting the growth and adhesion of the cells.

To meet some of these requirements, it is therefore desirable for the structure to be formed of nanofibres of an appropriate diameter and length, made from suitable materials.

The technique generally used for forming nanofibre structures is electrospinning, in which a polymer solution is introduced via a syringe into a high-potential electric field generated between the syringe and a collecting element. The solution flowing out of the syringe is lengthened, and therefore thinned, by the action of the electric field while the solvent evaporates as it travels between the syringe needle and the collecting element. In this way, polymeric fibres of extremely small diameter - of the order of tens or some hundreds of nanometres - are deposited on the collecting element.

Furthermore, by varying the parameters of the process as appropriate, the orientation of the nanofibres and the porosity of the nanofibre structure obtained can also be controlled and modified to a certain extent.

Nanofibre structures of this type are produced using biocompatible and bioabsorbable polymers such as polylactic acid (PLA), polyglycolic acid (PGA) and polycaprolactone (PCL).

As stated above, SAPs have the feature of assembling together when placed under suitable conditions, thus forming nanofibres having a very small diameter, about 5-10 nanometres, and a length of the order of a few tens of nanometres at most.

Interactions between SAP nanofibres generate a hydrogel with a high water content, which can be used in tissue or bone regeneration treatment, particularly when included in a nanofibre structure formed by electrospinning a bioabsorbable polymer, or can be used as a solution for injection.

In fact, the hydrogel formed from SAPs is not able to maintain a defined shape (it is not a solid), and therefore it cannot be used for forming nanofibre structures of the above-mentioned type (scaffolds) capable of effectively replacing the extracellular matrix.

In general, because the hydrogel formed by the SAPs cannot be given a defined shape, there are other drawbacks since it cannot be individually manipulated and always needs a supporting structure in order to be shaped as appropriate for the therapeutic treatment required.

The scientific literature (Acta Biomaterialia 7; (2011); 2526-2532) discloses a method for producing a nanofibre structure starting from a polyethylene oxide (PEO)-based electrospinning solution, within which a self-assembling peptide fraction is dispersed.

Nanofibres can thus be produced with a length and nanofibre structure giving them mechanical properties that make them individually manipulable.

In this structure, however, the percentage of SAPs proves to be extremely low compared with the base polymer, estimated to be about 1%, and consequently the positive effect of the SAPs in the structure is not maximised.

Furthermore, electrospinning a single SAP solution and another polymer generally leads to an intimate mixture of the two compounds, which will be more homogeneous the greater their chemical compatibility. Any separation between the two compounds could only be observed in cases where they were highly incompatible but, in any case, the two compounds would be arranged in the nanofibre in an entirely non-homogeneous and random manner.

An analogous nanofibre structure obtained by electrospinning a single solution of SAP and polycaprolactone is described in *"A structurally SAP nano-architecture by one step electrospinning"* (Gharaei et al. vol. 4, pages 5475-5485, 25 July 2016, Journal of Materials Chemistry*).*

### Definitions

The term self-assembling peptide or SAP refers to a family of oligopeptides, formed of a chain of 5 to 35 amino-acids, capable of assembling together under suitable conditions as a stable supra-molecular structure of nanometric dimensions.

The term "nanofibre" means fibres that have a mean diameter of the order of tens or hundreds of nanometres, certainly less than one micron.

### Description of the invention

The problem addressed by the present invention is that of providing a method for producing a nanofibre structure, and a nanofibre structure based on self-assembling peptides that overcome the drawbacks complained of above with reference to the prior art mentioned.

In particular, an aim of the present invention is to produce a nanofibre structure based on SAPs that are rigid enough to be individually manipulable. Another aim of the invention is to produce an SAP-based nanofibre structure that can be shaped at will.

Another aim of the invention is to produce a nanofibre structure formed of long nanofibres of self-assembling peptides alone.

Another aim of the invention is to provide a production method that is relatively simple and little expensive.

These and other aims are achieved by the present invention by means of a production method and a nanofibre structure in accordance to the appended claims.

In a first aspect thereof, the invention is aimed at a method for producing nanofibres based on self-assembling peptides using a co-electrospinning technique.

Preferably, this method comprises the steps of preparing a first solution of SAPs and a second solution of a coating polymer, and subjecting the first solution and the second solution to a co-electrospinning step, in which said first solution and said second solution are electrospun at the same time. Preferably, the second solution is electrospun around the first solution, so as to produce nanofibres with a central core formed of self-assembling peptides and an outer layer, formed of the coating polymer, surrounding the central core.

In this way, the SAPs present inside the nanofibre, and hence contained within a very small space defined by the outer layer, are prompted to assemble together as a lengthy, orderly nanofibre, similar to those of the outer layer of the nanofibre.

Indeed, the outer layer formed of the coating polymer performs the function of guide and containment, which favours the orderly process of self-assembly of the peptides.

In a second aspect thereof, the invention is aimed at an SAP-based nanofibre structure that can be produced by the method described with reference to the preceding aspect.

The nanofibre structure produced by this method advantageously has a defined, solid, individually manipulable form.

Furthermore, it can conveniently be shaped according to the requirements of the application, for example in the form of a membrane.

Thanks to these characteristics, it can also be applied directly to the tissues to be regenerated, without the need for another supporting structure.

Once this nanofibre structure is positioned on the region of the body to be treated, which may for example be an internal or superficial lesion or wound, a degradation process of the outer fibre layer carrying the SAPs within the nanofibres, on direct contact with the surrounding physiological environment, will start, such that SAPs can perform their beneficial regenerative function. The period of degradation of the outer layer depends, in general, on the type of coating polymer used, and this can therefore be selected as appropriate according to specific therapeutic requirements.

It should be noted that, following this degradation process, the nanofibres of the structure are formed substantially of SAPs only.

In a third aspect thereof, the invention relates to a medical device comprising the aforesaid nanofibre structure.

In at least one of the aspects indicated above, the present invention can also have at least one of the preferred features mentioned below.

In a preferred embodiment, at least 50% of the nanofibres based on self-assembling peptides that form the nanofibre structure are longer than 50 microns.

More preferably, at least 70% of said nanofibres are longer than 70 microns and 50% of the nanofibres are longer than 100 microns.

In one embodiment, nanofibres a few millimetres long and, even more preferably, equal to or longer than one centimetre, are present in the structure.

In one embodiment, the nanofibres have a mean diameter of between 50 and 800 nanometres; they preferably have a mean diameter of between 200 and 500 nanometres.

In this way, the nanofibre structure has mechanical properties suitable for maintaining its shape and being easy to manipulate.

Preferably, the first SAP-based solution is an aqueous solution comprising a fraction of SAPs of between 0.1% and 4% w/v (weight/volume) preferably between 0.5 and 2% w/v (weight/volume), and most preferably about 1% w/v.

The aqueous solution can contain only SAPs as a solute or, in other embodiments, the aqueous solution can also contain one or more salts.

In one embodiment the aqueous solution is a buffer solution and, for example, comprises suitable quantities of potassium hydrogen phosphate (KH₂PO₄) and/or sodium hydrogen phosphate (Na₂HPO₄) with sodium chloride (NaCl) and/or potassium chloride (KCI), to form a phosphate-buffered saline (PBS).

In a preferred embodiment, the SAPs are selected from a group comprising the following peptides, wherein, for each peptide, the reference name and the identification number of the amino-acid sequence given in the list of sequences attached to this description are provided:
EAK16-II (SEQ ID NO:1);
RADA16-I (SEQ ID NO:3);
RGDA16-I (SEQ ID NO:4);
RADA8-I (SEQ ID NO:5);
RAD16-II (SEQ ID NO:6);
RAD8-II (SEQ ID NO:7);
EAKA16-I (SEQ ID NO:8);
EAKA8-I (SEQ ID NO:9);
RAEA16-I (SEQ ID NO:10);
RAEA8-I (SEQ ID NO:11);
KADA16-I (SEQ ID NO:12);
KADA8-I (SEQ ID NO:13);
EAH16-II (SEQ ID NO:14);
EAH8-I (SEQ ID NO:15);
EFK16-II (SEQ ID NO:16);
EFK12-I (SEQ ID NO:17);
EFK8-II (SEQ ID NO:18);
ELK16-II (SEQ ID NO:19);
ELK8-II (SEQ ID NO:20);
EAK12 (SEQ ID NO:21);
EAK8-II (SEQ ID NO:22);
KAE16-IV (SEQ ID NO:23);
EAK16-IV (SEQ ID NO:24);
KLD12-I (SEQ ID NO:25);
KLE12-I (SEQ ID NO:26);
RAD16-IV (SEQ ID NO:27);
DAR16-IVa (SEQ ID NO:28);
DAR16-IV (SEQ ID NO:29);
DAR32-IV (SEQ ID NO:30);
EHK16 (SEQ ID NO:31);
EHK8-I (SEQ ID NO:32);
VE20 (SEQ ID NO:33);
RF20 (SEQ ID NO:34).

Most preferably, the SAPs are selected from the group consisting of EAK16-II (SEQ ID NO:1), RADA16-I (SEQ ID NO:3), RGDA16-I (SEQ ID NO:4), RAD16-II (SEQ ID NO:6), EAK12 (SEQ ID NO:21), EAK8-II (SEQ ID NO:22) and DAR16-IV (SEQ ID NO:29).

In a preferred version of the invention, the SAPs are duly functionalised by adding one or more growth factors or cell adhesion molecules, or else chemotactic factors, to the peptide chain.

Preferably, these functional groups are such that they do not deprive the SAPs of their ability to self-assemble.

Examples of growth factors that can be used in the present invention are: platelet-derived growth factor (PDGF), epidermal growth factor (EGF), insulin-like growth factor (IGF-1 and IGF-2), fibroblast growth factors (for example FGF1 and FGF24) and also vascular endothelial growth factor (VEGF).

Examples of cell adhesion molecules that can be used in the present invention are molecules that tend to bind to cell surface receptors, such as integrin. These include the oligopeptides RGD (arginylglycylaspartic acid), PHSRN (proline-histidine-serine-arginine-asparagine, SEQ ID NO:35) and GFOGER (glycine-phenylalanine-hydroxyproline-glycine-glutamate-arginine), as well as protein fragments containing these adhesion molecules, in particular fragments of fibronectines or collagen.

A preferred example of SAP functionalised with a cell adhesion molecule is RGD-EAK16-II, whose amino-acid sequence is shown in the sequence list as (SEQ ID NO:2).

Preferably, the second polymer-based coating solution is an aqueous solution comprising a fraction of coating polymer of between 3% and 10% w/v (weight/volume), and most preferably a fraction of about 5% w/v.

Preferably, the coating polymer is biocompatible and, most preferably, is bioabsorbable.

Preferably, the coating polymer is selected from the group consisting of polyethylene glycol, polyoxyethylene, polyvinyl alcohols, polyvinylpyrrolidone (PVP), polycaprolactone (PCL), polyacrylic acid, albumin, polysaccharides and derivatives thereof, examples of which are pectins, xanthan, hyaluronic acid, chitosan, dextran, carrageenan, guar gum, cellulose ethers and starch.

Even more preferably, the coating polymer is selected from the group formed of polyethylene glycol (PEG), chitosan, polyglycolic acid (PGA), polylactic acid (PLA), hyaluronic acid (HA), polyvinylpyrrolidone (PVP), polycaprolactone (PCL), and salts, mixtures and copolymers thereof.

In one embodiment, it is provided for the coating polymer forming the outer layer of the nanofibre to break down after the co-electrospinning step.

This will allow a nanofibre structure to be produced, formed solely of SAP nanofibres; said structure can be used as it is on the region to be treated and may be able to perform its regenerative action immediately.

Preferably, the co-electrospinning process is carried out by means of a syringe having a needle with at least two concentric channels.

Preferably, the SAP solution is caused to flow through an inner channel of said concentric-channel needle, while the solution with the coating polymer is caused to flow through an outer channel of the concentric-channel needle.

In this way, until they exit the needle, the two solutions never come into contact, so that the SAP leaves the needle in an inner position of the outgoing flow and the outer polymer leaves the needle in a coaxial, outer position of the outgoing flow.

Once they leave the needle, the SAP and coating polymer still remain substantially separate, since the solvent evaporates very quickly, greatly slowing down the possibility of mixing and, in fact, locking the reciprocal positioning of the two compounds. Said two compounds of the nanofibre thus produced are therefore, in one case (SAP), substantially concentrated inside and, in the other case (coating polymer), substantially concentrated on the outer layer.

Preferably, the inner channel has a diameter of between 0.5 and 1 mm, while the outer channel has a diameter of between 1.5 and 2 mm.

Preferably, the co-electrospinning is carried out in an electric field generated by a potential difference of between 10 and 25 kV applied between the syringe and a collecting element.

In a preferred version of the invention, the nanofibres are deposited on the collecting element in the form of a membrane.

The thickness of the membrane can be varied at will, for example by adjusting the electrospinning period, depending on the desired characteristics of the nanofibre structure. In a preferred embodiment, the membrane is between 200 and 600 microns thick.

In one embodiment, the nanofibres in the membrane, or more generally in the nanofibre structure, are intertwined at random, in the form of a nonwoven fabric.

In another embodiment of the invention the nanofibres are substantially aligned with one another.

In this embodiment, the nanofibre structure produced in this way is advantageously used for producing hollow conduits, preferably having the size of the small vessels (vessels with a diameter of less than 3 mm), or as a hollow-conduit coating.

This structure can be produced by using an electrospinning device whose collecting element is formed of a cylindrical collector operating at a suitable rotation speed to allow the nanofibres leaving the needle to be wound, possibly stretching them, but without breaking them.

In another embodiment of the invention the nanofibres are twisted together to form a yarn.

Accordingly, these yarns can be used to produce a suture stitch.

This structure can be produced by using an electrospinning device whose collecting element is formed of a rod-shaped collector extending towards the needle and operating in rotation about its axis so as to twist the nanofibres extending from the collector to the needle.

### Brief description of the drawings

The characteristics and advantages of the invention will be more clearly apparent from the following detailed description of a preferred exemplary embodiment thereof, illustrated for information and non-restrictively, with reference to the appended figures, in which:
- Fig. 1 shows a diagrammatic view of a piece of co-electrospinning equipment designed to work according to the method of the present invention,
- Fig. 2 and 3 are photos taken by electron microscope, respectively at 2000 magnification and 10000 magnification, of a nanofibre structure obtained according to the present invention,
- Fig. 4 to 6 are photos of the confocal microscopic analysis of the nanofibre structure in Fig. 2 and 3, and
- Fig. 7 is a photo taken by electron microscope of a nanofibre structure obtained according to a variant embodiment of the present invention.

### Preferred embodiment of the invention

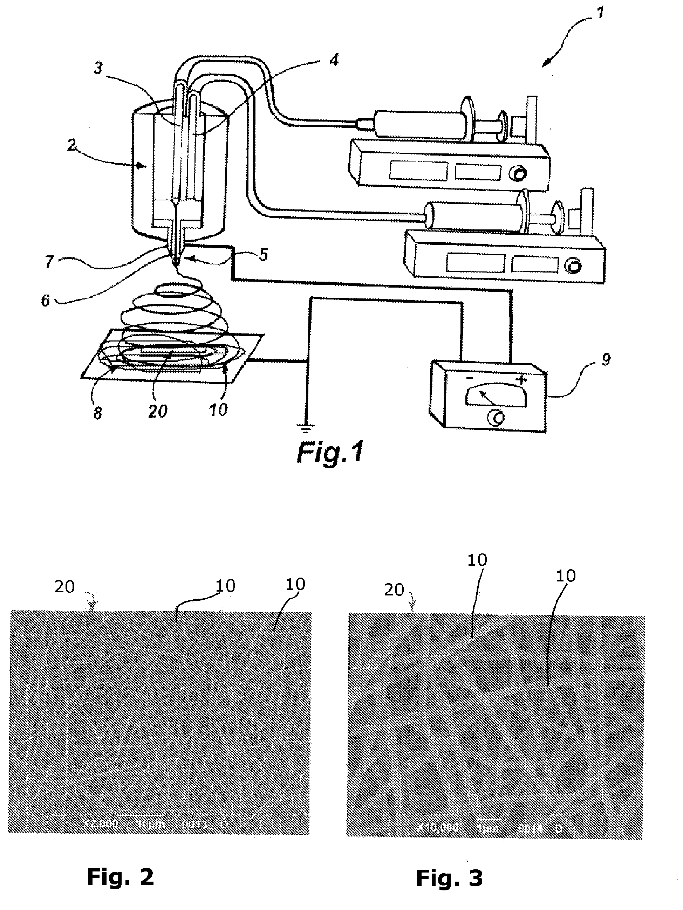

Fig. 1 shows diagrammatically a co-electrospinning equipment, indicated by 1 overall, designed to operate according to the method of the present invention. The equipment 1 comprises a syringe 2 having a first chamber 3 and a second chamber 4, not communicating between each other, and a needle 5, connected to both chambers 3 and 4.

The needle 5 comprises an inner channel 6, connected to the first chamber 3, and an outer channel 7, extending coaxially to the inner channel 6 but not communicating with it, which is connected to the second chamber 4.

The first and the second chamber 3, 4 can be positioned inside one another or can be separate and connected respectively to the inner channel 6 and the outer channel 7 by means of suitable connecting conduits.

The needle 5 points towards a collecting element 8, positioned a suitable distance away, on which the nanofibre material leaving the needle 5 is deposited.

The collecting element is preferably made of metallic material, for example it can be formed of an aluminium sheet.

Equipment 1 also comprises a voltage source 9, designed to generate an electric field between the syringe 2 and the collecting element 8, which have a predefined difference in potential.

A first, SAP-based solution is put in the first chamber 3, while a second, coating polymer-based solution is put in the second chamber 4.

Once the voltage source 9 has been actuated, thus forming the electric field between syringe 2 and collecting element 8, the first and second solution are delivered from the respective chambers 3 and 4 through the inner channel 6 and, respectively, the outer channel 7 of the needle 5.

In this way the two solutions leave the needle 5 in a coaxial position, with the first solution inside the second solution, which surrounds it externally.

Through the action of the electric field the two solutions leaving the needle 5 form a respective Taylor cone and are prolonged towards the collecting element 8, thinning until they reach a diameter of the order of a few hundreds of nanometres.

The two solutions maintain the same spatial positioning even once they have left the needle 5, such that, as the solvent evaporates, a nanofibre 10 is formed with a central core 11, formed of the SAPs, surrounded by an outer layer 12, formed of the coating polymer.

The nanofibres 10 deposited on the collecting element 8 form a nanofibre structure 20, also a subject-matter of the present invention, which can, for example, take the form of a membrane.

The nanofibres can be oriented at random, to form a nonwoven fabric mat or, by varying the processing conditions as appropriate, they can be oriented parallel to a reference direction, or else can be twisted together to form a yarn.

The deposition of nanofibres is stopped when the nanofibre structure reaches the desired thickness, depending on the specific therapeutic applications for which it is intended.

### Example

First of all, the following were prepared: an adequate amount of RGD-EAK16-II, a self-assembling peptide, in which the amino-acid tail RGD, an adhesion molecule, was added to the SAP EAK-16-II in a terminal position, so as to form the overall structure SEQ ID NO:2.

Co-electrospinning equipment was provided, like that described with reference to Fig. 1, in which the needle comprised an inner channel with a diameter of 21 Gauge (about 0.8 mm) and an outer channel with a diameter of 15 Gauge (about 1.8 mm) communicating respectively with a first chamber and a second chamber of a syringe.

The following were also prepared: a first aqueous solution of RGD-EAK16-II at 1% w/v, introduced into the first chamber of the syringe, and a second solution of PEG (polyethylene glycol - 90 kDalton, Sigma Aldrich) at 5% w/v, introduced into the second chamber of the syringe.

Then the two solutions were co-electrospun at ambient temperature in an electric field generated by a potential difference between the syringe and a collecting element formed of an aluminium sheet, of about 18 kV.

The first solution was delivered by the first channel of the needle at a flow rate of about 0.2 mL/h, while the second solution was delivered by the second channel of the needle at a flow rate of about 0.8 mL/h.

The nanofibres produced were deposited on the aluminium sheet, forming a membrane of nanofibres tangled at random, to reach a thickness of about 400 microns.

The membrane thus obtained, having been easily detached from the aluminium sheet, was observed under an electron microscope.

Fig. 2 and 3 show the SEM photographs of a representative portion of the membrane in which the nanofibres produced by the method are visible at various magnifications.

The nanofibre structure is substantially free of defects, with nanofibres of a diameter of about 300 nanometres and a length of hundreds of microns, with several fibre lengths ranging from some millimetres, up to a few centimetres. The coaxial structure of the nanofibres was demonstrated by a confocal microscopy analysis in which the materials of the nanofibres (PEG and RGD-EAK16-II), previously marked respectively with fluorescein isothiocyanate and rhodamine, were visualised with different colours.

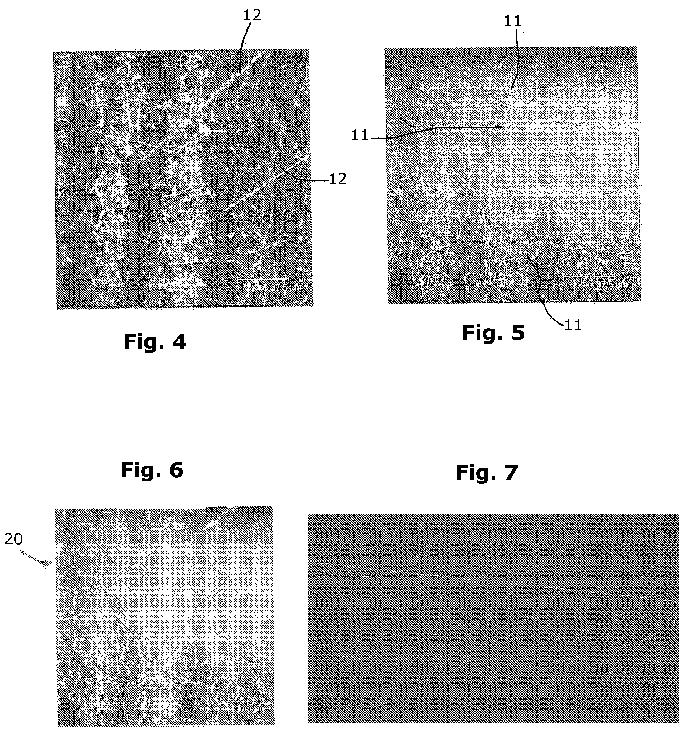

Fig. 4 shows an image revealing only the PEG component of the structure, while Fig. 5 shows an image revealing only the SAP component of the structure.

The image in Fig. 6, showing both colours with which the PEG and the SAP were marked, reveal both the PEG structure and the SAP structure.

By analysing the images, it is clear that a large part of the PEG nanofibres correspond to respective SAP nanofibres.

The nanofibre structure was also subjected to mechanical strength tests, using a machine for measuring biaxial planar deformation depending on the load applied.

Taking into account a structure porosity of about 80%, the test revealed a modulus of elasticity of about 1.28 MPa and an ultimate tensile strength of about 0.68 MPa, substantially comparable to random nanofibre structures based only on PEG.

The nanofibre structure obtained, although friable, has a shape of its own and can be manipulated individually, for example by using tweezers, in order to subject it to any further processing, for example to shape it appropriately in a medical device of a suitable shape for the specific intended use.

Fig. 7 shows an electron microscope photograph of a nanofibre structure produced by the method described above, in which the nanofibres were collected on a rotating cylindrical collector. In this way, as clearly visible from the photo, the nanofibres are substantially aligned in a preferential direction.

The present invention therefore resolves the problem complained of above with regard to the prior art mentioned.

## Claims

1. A method for producing nanofibres based on self-assembling peptides (SAP), comprising:
- preparing a first solution based on self-assembling peptides;
- preparing a second solution based on a coating polymer;
- subjecting said first solution and said second solution to a co-electrospinning step, in which said first solution and said second solution are electrospun at the same time, said second solution being electrospun around said first solution so as to produce nanofibres having a central core formed by said self-assembling peptides and an outer layer, which surrounds said core and is formed by said coating polymer.

2. The method according to claim 1, wherein said self-assembling peptides are selected from the group consisting of SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33 and SEQ ID NO:34.

3. The method according to claim 2, wherein said self-assembling peptides are functionalised with a growth factor or a cell adhesion molecule or a chemotactic factor.

4. The method according to any one of the preceding claims, wherein said coating polymer is selected from the group consisting of polyethylene glycol, polyoxyethylene, polyvinyl alcohols, polyvinylpyrrolidone (PVP), polycaprolactone (PCL), polyacrylic acid, albumin, polysaccharides and derivatives thereof.

5. The method according to claims 3 and 4, wherein said functionalised self-assembling peptide is SEQ ID NO:2 and said coating polymer is polyethylene glycol (PEG).

6. The method according to any one of the preceding claims, wherein said co-electrospinning step is performed using a needle with at least two concentric channels and said first solution is caused to flow through an inner channel of said needle while said second solution is caused to flow through an outer channel of said needle.

7. A nanofibre structure based on self-assembling peptides, **characterised in that** said structure comprises nanofibres having a core formed by said self-assembling peptides and an outer layer, which surrounds said core and is formed by a coating polymer.

8. The structure according to claim 7, wherein at least 50% of said nanofibres have a length greater than 50 microns.

9. The structure according to claim 8, wherein at least 70% of said nanofibres have a length greater than 70 microns and 50% of said nanofibres have a length greater than 100 microns.

10. The structure according to any one of claims 7 to 9, wherein said self-assembling peptides are selected from the group consisting of SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34.

11. The structure according to claim 10, wherein said self-assembling peptides are functionalised with a growth factor or a cell adhesion molecule or a chemotactic factor.

12. The structure according to any one of claims 7 to 11, wherein said coating polymer is selected from the group consisting of polyethylene glycol, polyoxyethylene, polyvinyl alcohols, polyvinylpyrrolidone (PVP), polycaprolactone (PCL), polyacrylic acid, albumin, polysaccharides and derivatives thereof.

13. The structure according to any one of claims 7 to 12, wherein said nanofibres are intertwined at random, in the form of a nonwoven fabric.

14. The structure according to any one of claims 7 to 12, wherein said nanofibres are substantially aligned with one another.

15. The structure according to any one of claims 7 to 12, wherein said nanofibres are twisted together to form a yarn.

16. A medical device having a nanofibre structure according to any one of claims 7 to 15.

17. The medical device according to claim 16 for use in tissue or bone regeneration treatment.

## Patentansprüche

1. Verfahren zur Herstellung von Nanofasern basierend auf selbstassemblierenden Peptiden (SAP),
umfassend:
- Herstellen einer ersten Lösung basierend auf selbstassemblierenden Peptiden;
- Herstellen einer zweiten Lösung basierend auf einem Beschichtungspolymer;
- Unterziehen der ersten Lösung und der zweiten Lösung einem Co-Elektrospinnschritt, bei dem die erste Lösung und die zweite Lösung gleichzeitig elektrogesponnen werden, wobei die zweite Lösung um die erste Lösung herum elektrogesponnen wird, um Nanofasern mit einem zentralen Kern, der durch die selbstassemblierenden Peptide gebildet wird, und einer äußeren Schicht, die den Kern umgibt und durch das Beschichtungspolymer gebildet wird, herzustellen.

2. Verfahren nach Anspruch 1, wobei die selbstassemblierenden Peptide aus der Gruppe bestehend aus SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO: 14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO: 24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33 und SEQ ID NO:34 ausgewählt sind.

3. Verfahren nach Anspruch 2, wobei die selbstassemblierenden Peptide mit einem Wachstumsfaktor oder einem Zelladhäsionsmolekül oder einem chemotaktischen Faktor funktionalisiert sind.

4. Verfahren nach einem der vorhergehenden Ansprüche, wobei das Beschichtungspolymer aus der Gruppe bestehend aus Polyethylenglykol, Polyoxyethylen, Polyvinylalkoholen, Polyvinylpyrrolidon (PVP), Polycaprolacton (PCL), Polyacrylsäure, Albumin, Polysacchariden und Derivaten davon ausgewählt ist.

5. Verfahren nach den Ansprüchen 3 und 4, wobei das funktionalisierte selbstassemblierende Peptid SEQ ID NO:2 ist und das Beschichtungspolymer Polyethylenglykol (PEG) ist.

6. Verfahren nach einem der vorhergehenden Ansprüche, wobei der Schritt des Co-Elektrospinnens unter Verwendung einer Nadel mit zumindest zwei konzentrischen Kanälen durchgeführt wird und die erste Lösung durch einen inneren Kanal der Nadel fließen gelassen wird, während die zweite Lösung durch einen äußeren Kanal der Nadel fließen gelassen wird.

7. Nanofaserstruktur basierend auf selbstassemblierenden Peptiden, **dadurch gekennzeichnet, dass** die Struktur Nanofasern mit einem Kern, der aus den selbstassemblierenden Peptiden gebildet ist, und eine äußere Schicht umfasst, die den Kern umgibt und aus einem Beschichtungspolymer gebildet ist.

8. Struktur nach Anspruch 7, wobei zumindest 50 % der Nanofasern eine Länge von mehr als 50 Mikrometer aufweisen.

9. Struktur nach Anspruch 8, wobei zumindest 70 % der Nanofasern eine Länge von mehr als 70 Mikrometer aufweisen und 50 % der Nanofasern eine Länge von mehr als 100 Mikrometer aufweisen.

10. Struktur nach einem der Ansprüche 7 bis 9, wobei die selbstassemblierenden Peptide aus der Gruppe bestehend aus SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO: 14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO: 24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33 und SEQ ID NO:34 ausgewählt sind.

11. Struktur nach Anspruch 10, wobei die selbstassemblierenden Peptide mit einem Wachstumsfaktor oder einem Zelladhäsionsmolekül oder einem chemotaktischen Faktor funktionalisiert sind.

12. Struktur nach einem der Ansprüche 7 bis 11, wobei das Beschichtungspolymer aus der Gruppe bestehend aus Polyethylenglykol, Polyoxyethylen, Polyvinylalkoholen, Polyvinylpyrrolidon (PVP), Polycaprolacton (PCL), Polyacrylsäure, Albumin, Polysacchariden und Derivaten davon ausgewählt ist.

13. Struktur nach einem der Ansprüche 7 bis 12, wobei die Nanofasern in Form eines Vliesstoffs willkürlich miteinander verflochten sind.

14. Struktur nach einem der Ansprüche 7 bis 12, wobei die Nanofasern im Wesentlichen zueinander ausgerichtet sind.

15. Struktur nach einem der Ansprüche 7 bis 12, wobei die Nanofasern miteinander verdrillt sind, um ein Garn zu bilden.

16. Medizinische Vorrichtung mit einer Nanofaserstruktur nach einem der Ansprüche 7 bis 15.

17. Medizinische Vorrichtung nach Anspruch 16 zur Verwendung bei der Gewebe-oder Knochenregenerationsbehandlung.

## Revendications

1. Procédé de production de nanofibres à base de peptides auto-assemblés (SAP), comprenant :
- la préparation d'une première solution à base de peptides auto-assemblés ;
- la préparation d'une seconde solution à base d'un polymère de revêtement ;
- la soumission de ladite première solution et de ladite seconde solution à une étape de co-électrofilage, dans lequel ladite première solution et ladite seconde solution sont électrofilées en même temps, ladite seconde solution étant électrofilée autour de ladite première solution de manière à produire des nanofibres ayant un noyau central formé par lesdits peptides auto-assemblés et une couche externe, qui entoure ledit noyau et est formée par ledit polymère de revêtement.

2. Procédé selon la revendication 1, dans lequel lesdits peptides auto-assemblés sont choisis dans le groupe constitué par SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33 et SEQ ID NO:34.

3. Procédé selon la revendication 2, dans lequel lesdits peptides auto-assemblés sont fonctionnalisés avec un facteur de croissance, une molécule d'adhésion cellulaire ou un facteur chimiotactique.

4. Procédé selon l'une quelconque des revendications précédentes, dans lequel ledit polymère de revêtement est choisi dans le groupe constitué par le polyéthylène glycol, le polyoxyéthylène, les alcools polyvinyliques, la polyvinylpyrrolidone (PVP), la polycaprolactone (PCL), l'acide polyacrylique, l'albumine, les polysaccharides et leurs dérivés.

5. Procédé selon les revendications 3 et 4, dans lequel ledit peptide auto-assemblé fonctionnalisé est SEQ ID NO:2 et ledit polymère de revêtement est le polyéthylène glycol (PEG).

6. Procédé selon l'une quelconque des revendications précédentes, dans lequel ladite étape de co-électrofilage est réalisée à l'aide d'une aiguille comportant au moins deux canaux concentriques et dans lequel ladite première solution est amenée à s'écouler dans un canal interne de ladite aiguille tandis que ladite seconde solution est amenée à s'écouler dans un canal externe de ladite aiguille.

7. Structure de nanofibres à base de peptides auto-assemblés, **caractérisée en ce que** ladite structure comprend des nanofibres ayant un noyau formé par lesdits peptides auto-assemblés et une couche extérieure, qui entoure ledit noyau et est formée par un polymère de revêtement.

8. Structure selon la revendication 7, dans laquelle au moins 50 % desdites nanofibres ont une longueur supérieure à 50 microns.

9. Structure selon la revendication 8, dans laquelle au moins 70 % desdites nanofibres ont une longueur supérieure à 70 microns et 50 % desdites nanofibres ont une longueur supérieure à 100 microns.

10. Structure selon l'une quelconque des revendications 7 à 9, dans laquelle lesdits peptides auto-assemblés sont choisis dans le groupe constitué par SEQ ID NO:1, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34.

11. Structure selon la revendication 10, dans laquelle lesdits peptides auto-assemblés sont fonctionnalisés avec un facteur de croissance, une molécule d'adhésion cellulaire ou un facteur chimiotactique.

12. Structure selon l'une quelconque des revendications 7 à 11, dans laquelle ledit polymère de revêtement est choisi dans le groupe constitué par le polyéthylène glycol, le polyoxyéthylène, les alcools polyvinyliques, la polyvinylpyrrolidone (PVP), la polycaprolactone (PCL), l'acide polyacrylique, l'albumine, les polysaccharides et leurs dérivés.

13. Structure selon l'une quelconque des revendications 7 à 12, dans laquelle lesdites nanofibres sont entrelacées de manière aléatoire, sous la forme d'un tissu non tissé.

14. Structure selon l'une quelconque des revendications 7 à 12, dans laquelle lesdites nanofibres sont sensiblement alignées les unes sur les autres.

15. Structure selon l'une quelconque des revendications 7 à 12, dans laquelle lesdites nanofibres sont torsadées ensemble pour former un fil.

16. Dispositif médical ayant une structure en nanofibres selon l'une quelconque des revendications 7 à 15.

17. Dispositif médical selon la revendication 16 pour une utilisation dans un traitement de régénération tissulaire ou osseuse.
